# FASCICULE DE BREVET EUROPEEN

(11) **EP 0 597 069 B1**
(45) Date de publication et mention de la délivrance du brevet: **12.08.1998**
(21) Numéro de dépôt: 93911791.7
(22) Date de dépôt: 03.05.1993
(51) Int. Cl.: C12P 7/04, C12P 7/24

(54) **Procédé enzymatique pour la préparation de n-hexanal, 3-(Z)-hexén-1-al, ou 2-(E)-hexén-1-al, ou des alcools correspondants mettant en oeuvre une lipoxygénase de soja et une lyase de goyave**
Enzymatisches Verfahren zur Herstellung von n-Hexanal, 3-(Z)-Hexen-1-al, 2-(E)-hexen-1-al, oder entsprechenden Alkoholen mittels einer Soja-Lipoxygenase und einer Guave-Lyase
Enzymic method of producing n-hexanal, 3-(Z)-hexen-1-al, 2-(E)-hexen-1-al, or corresponding alcohols using a soja lipoxygenase and a guava lyase

(30) Priorité: 26.05.1992 CH 1693/92; 28.07.1992 CH 2372/92; 01.10.1992 CH 3075/92
(43) Date de publication de la demande: 18.05.1994
(73) Titulaire: FIRMENICH SA, 1211 Genève 8 (CH); MULLER, Bernard, CH-1292 Chambésy (CH); GAUTIER, Antoine, F-74160 Archamps (FR); DEAN, Christopher, CH-1212 Grand-Lancy (CH); KUHN, Jean-Charles, CH-1203 Genève (CH)
(72) Inventeur: MULLER, Bernard, CH-1292 Chambésy (CH); GAUTIER, Antoine, F-74160 Archamps (FR); DEAN, Christopher, CH-1212 Grand-Lancy (CH); KUHN, Jean-Charles, CH-1203 Genève (CH)
(74) Mandataire: Salvaterra-Garcia, Maria de Lurdes
(86) Numéro de dépôt international: EP9301057
(87) Numéro de publication internationale: WO9324644

(56) Documents cités:
- CHEMISTRY AND PHYSICS OF LIPIDS vol. 44, no. 2-4, Septembre 1987, pages 341 - 361 AKIKAZU HATANAKA ET AL. 'Biosynthetic pathway for C6-aldehydes formation from linolenic acid in green leaves'
- AGRICULTURAL AND BIOLOGICAL CHEMISTRY. vol. 43, no. 5, 1979, TOKYO JP pages 969 - 980 JIRO SEKIYA ET AL. 'Volatile C6-aldehyde formation via Hydroperoxides from C18-unsaturated fatty acids in etiolated alfalfa and cucumber seedlings'
- CHEMICAL ABSTRACTS, vol. 111, 1989, Columbus, Ohio, US; abstract no. 74853n, HATANAKA, AKIKAZU ET AL. 'Biogeneration of green odor (I). Enzymic oxygen-active-cleavage reaction of linolenic acid in leaves.' page 455 ;colonne L ;

## Description

### Domaine technique

La présente invention a trait au domaine de la synthèse organique et plus particulièrement elle a pour objet un procédé enzymatique permettant de préparer des aldéhydes et des alcools ayant six atomes de carbone, notamment les n-hexanal, 3-(Z)-hexén-1-al, 2-(E)-hexén-1-al et leurs alcools correspondants. Il s'agit de composés oxygénés d'emploi courant dans l'industrie des arômes, notamment pour leurs caractères organoleptiques de type fruité et vert. Connus pour être des constituants de plusieurs arômes d'origine naturelle, nombreuses sont les études décrites dans la littérature qui rapportent des voies de synthèse variées de ces composés.

Compte tenu des normes législatives actuellement en vigueur dans la plupart des pays relatives à l'utilisation d'additifs alimentaires, il devient impératif de disposer des aldéhydes et alcools susmentionnés en une qualité qui puisse satisfaire les critères établis quant au caractère naturel de leur origine. Quoiqu'en principe l'on puisse envisager d'obtenir ces composés par extraction des produits d'origine naturelle les renfermant, il apparaît à l'évidence que, vu leurs proportions, une telle méthode n'est guère économique.

### Technique antérieure

Parmi les études décrites dans la littérature ayant trait à des voies de synthèse des composés susmentionnés, il convient de citer celui décrit dans la demande de brevet française n^{º} 2652587, publiée le 5 avril 1991, qui fait état d'un procédé biologique de préparation du 3-(Z)-hexén-1-ol à partir d'acide gras insaturé. Le procédé décrit a recours à l'action combinée d'un système enzymatique naturel introduit sous forme de fanes de radis ou de Rumex et d'une levure apte à promouvoir la réduction du 3-(Z)-hexén-1-al en 3-(Z)-hexén-1-ol. Selon les auteurs, un tel procédé permettait d'améliorer les rendements en l'alcool désiré par rapport aux résultats obtenus dans les procédés biologiques pratiqués précédemment, tels par exemple ceux décrits dans les brevets US 4,769,243 ou 4,806,379.

Quoique nous ayons pu vérifier le bien-fondé d'une telle affirmation, des études que nous avions menées en parallèle nous ont montré qu'il était possible de dépasser sensiblement les taux de rendement indiqués dans la demande de brevet citée, grâce au procédé qui fait l'objet de la présente invention.

### Exposé de l'invention

La présente invention a pour objet de fournir une méthode de préparation d'aldéhydes et alcools ayant 6 atomes de carbone qui satisfait d'une part à l'exigence légale d'origine naturelle et d'autre part aux critères de rentabilité industrielle.

En effet, nous avons découvert qu'il était désormais possible d'obtenir les n-hexanal, 3-(Z)-hexén-1-al et 2-(E)-hexén-1-al, ainsi que leurs alcools correspondants, par un procédé enzymatique à partir de l'acide linoléique ou linolénique, ou d'un précurseur d'origine naturelle dudit acide sous forme d'hydrolysat d'huile végétale, lequel procédé est caractérisé en ce qu'on effectue les étapes réactionnelles distinctes suivantes:
a) on soumet soit l'acide linoléique, soit l'acide linolénique, ou ledit précurseur sous forme d'hydrolysat d'huile végétale, à l'action d'une lipoxygénase du soja dans un milieu réactionnel approprié, pour obtenir soit l'acide 13-hydropéroxy-octadéca-9,11-diénoïque ("C₁₃HDPO"), soit l'acide 13-hydropéroxy-octadéca-9,11,15-triénoïque ("C₁₃HDPOT") ;
b) on transforme soit le "C₁₃HDPO", soit le "C₁₃HDPOT", ainsi formé soit en n-hexanal, soit en 3-(Z)-hexén-1-al, à l'aide d'une lyase de la goyave;
c) on réduit soit le n-hexanal, soit le 3-(Z)-hexén-1-al, obtenu au moyen d'une levure pour fournir soit le n-hexanol, soit le 3-(Z)-hexén-1-ol;
ou
c') le cas échéant, on isomérise le 3-(Z)-hexén-1-al dans les conditions de température et pH appropriées pour obtenir le 2-(E)-hexén-1-al, et
d) on réduit le 2-(E)-hexén-1-al obtenu au moyen d'une levure pour fournir le 2-(E)-hexén-1-ol.

Tel qu'il apparaît de ce qui précède, les aldéhydes sont obtenus en arrêtant le procédé à l'étape b) ou c'), avant donc d'entamer leur réduction par la levure.

Les produits désirés obtenus peuvent être séparés du milieu réactionnel par une étape supplémentaire qui consiste en un entraînement à la vapeur d'eau suivi d'une extraction à l'aide d'un solvant organique inerte.

Dans le cas de l'hexanal, l'entraînement à la vapeur d'eau permet l'obtention de deux couches bien distinctes de distillat, l'hexanal pouvant ainsi être séparé sans avoir recours à une extraction.

D'autre part, dans le cas du 3-(Z)-hexén-1-al, les meilleurs rendements sont obtenus par extraction avec un solvant organique inerte, sans entraînement à la vapeur d'eau.

Selon un mode préférentiel du procédé de l'invention, l'action enzymatique de lipoxygénase est exercée par de la farine de soja, laquelle est ajoutée au milieu réactionnel sous atmosphère d'oxygène. C'est ainsi que l'on obtient soit un hydropéroxyde de l'acide linoléique ou acide 13-hydropéroxyoctadéca-9,11-diénoïque (ci-après défini comme "C₁₃HDPO"), soit un hydropéroxyde de l'acide linolénique ou acide 13-hydropéroxy-octadéca-9,11,15-triénoïque (ci-après défini comme "C₁₃HDPOT"). Ces acides sont ensuite transformés soit en n-hexanal, soit en 3-(Z)-hexén-1-al sous l'action d'une lyase. Dans la pratique, il nous est apparu convenable de soumettre soit le "C₁₃HDPO", soit le "C₁₃HDPOT", à l'action d'un homogénat de goyave. L'homogénat de goyave est obtenu simplement par traitement des fruits dans un broyeur industriel ordinaire sans séparation préalable des parties solides. La transformation des aldéhydes ainsi obtenus en les alcools correspondants, à savoir le n-hexanol et le 3-(Z)-hexén-1-ol, se fait à l'aide d'une levure et, de préférence, on emploie une levure du type Saccharomyces cerevisiae.

Lorsqu'on désire obtenir l'alcool directement, il convient de soumettre soit le "C₁₃HDPO", soit le "C₁₃HDPOT", à l'action combinée d'un homogénat de goyave et de levure boulangère.

Comme mentionné plus haut, on peut utiliser comme produit de départ soit de l'acide linolénique, soit de l'acide linoléique, ou un de leurs précurseurs naturels et, à ce titre, nous avons porté notre choix soit sur l'huile de lin, soit sur l'huile de tournesol. Dans ce cas il faut bien entendu procéder au préalable à l'hydrolyse de ces huiles, laquelle peut s'effectuer de la façon suivante.

L'huile de lin, respectivement de tournesol, commerciale est traitée au reflux avec une solution aqueuse alcaline, par exemple d'hydroxyde de sodium, dans un alcool, par exemple l'éthanol ou le propylène glycol. Alternativement, l'huile de lin, respectivement de tournesol, peut être soumise à l'action enzymatique d'une lipase.

Un tel traitement permet une hydrolyse complète de l'huile et la solution résultante est utilisée directement pour la réaction avec le système enzymatique de lipoxygénase pour fournir le "C₁₃ HDPO", respectivement le "C₁₃HDPOT". Cette étape de la réaction est effectuée de préférence à un pH compris entre environ 9,0 et 9,5. Nous avons pu observer qu'une telle valeur de pH était critique pour l'obtention des rendements les meilleurs en hydropéroxyde, un phénomène qui est très probablement à mettre en relation avec l'activité de la lipoxygénase de la farine de soja.

D'autre part, nous avons également observé que la température pouvait jouer un rôle déterminant dans le bon déroulement de cette étape du procédé, en ce sens qu'une température préférentielle devait se situer au voisinage de la température ambiante et qu'il était déconseillé de conduire la réaction à des températures dépassant 30-35°C, peut être en raison d'un réarrangement de l'hydropéroxyde formé et la formation de produits secondaires non désirés.

A titre d'agent de lipoxygénase, nous avons eu recours à une farine de soja broyée immédiatement avant son emploi à partir de grains de soja. Alternativement, on pourrait avoir recours à une solution obtenue par traitement de cette farine avec de l'hexane, suivi d'un mélange avec une solution de borax et centrifugation.

En ce qui concerne les étapes suivantes b) et c) du procédé, nous avons également observé que l'obtention des rendements les meilleurs dépendait de l'acidité du milieu réactionnel, sans que cela toutefois soit aussi critique que dans l'étape précédente. En effet, les essais qui ont mené à la présente invention ont montré qu'on pouvait effectuer la réaction à une valeur du pH comprise entre environ 7,0 et 9,0, de préférence au voisinage de 8,0.

Comme il est indiqué plus haut, lorsqu'on désire obtenir le n-hexanol ou le 3-(Z)-hexén-1-ol, on peut procéder à l'exécution des deux étapes b) et c) de lyase et respectivement de réduction sans isolement du n-hexanal, respectivement 3-(Z)-hexén-1-al formé.

Pour obtenir le 2-(E)-hexén-1-al et le 2-(E)-hexén-1-ol, on isomérise d'abord le 3-(Z)-hexén-1-al obtenu selon l'étape b). Le rendement et la durée de cette réaction d'isomérisation se sont avérés être plus dépendants de la température que de l'acidité du milieu. Bien que la réaction puisse être conduite à des températures variant entre environ 40° et 80°C, les meilleurs rendements ont été obtenus à des températures de l'ordre de 50°C et à des pH légèrement acides, par exemple au voisinage de 6,5. Ce dernier peut être obtenu à l'aide d'acide citrique.

Après l'isomérisation totale du 3-(Z)-hexén-1-al en 2-(E)-hexén-1-al, ce dernier peut être transformé en 2-(E)-hexén-1-ol à l'aide de levure boulangère.

Le procédé de l'invention peut être illustré par le schéma suivant:

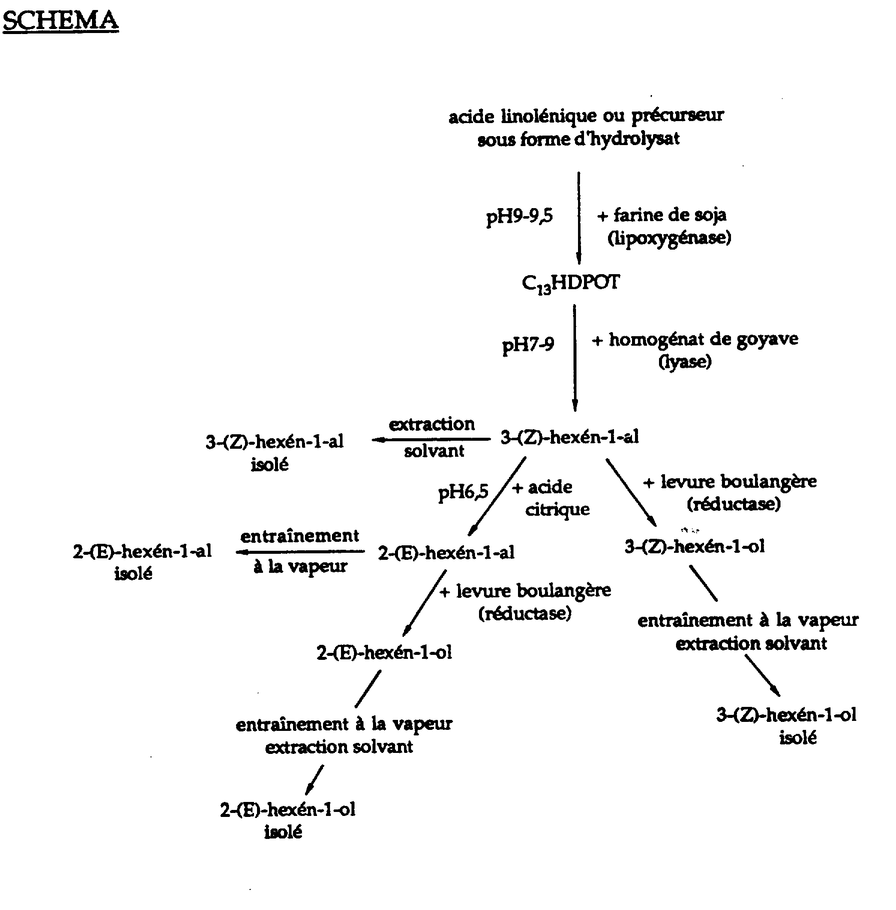

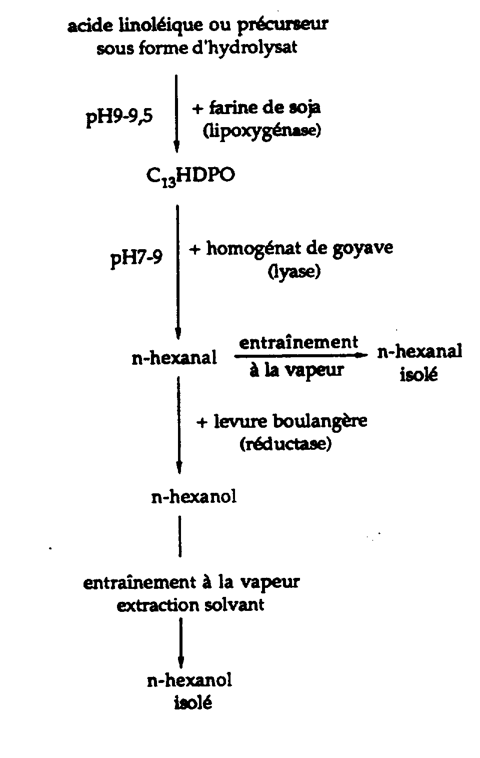

Par la mise en oeuvre du procédé de l'invention, nous avons obtenu le n-hexanal dans des concentrations proches de 5 g/kg de mélange réactionnel. Il en va de même avec le n-hexanol, ce qui représente un rendement global d'environ 36%.

De même, le 3-(Z)-hexén-1-ol a pu être obtenu dans des concentrations de l'ordre de 4,2 g/kg de mélange réactionnel, ce qui représente un rendement bien supérieur à celui qui avait été observé par la mise en oeuvre des procédés de l'art antérieur [0,050 g/kg].

Quant au 2-(E)-hexén-1-al, il a été obtenu dans des concentrations proches de 1,5 g/kg de mélange réactionnel, ce qui représente un rendement global d'environ 20%. Le 2-(E)-hexén-1-ol a lui été obtenu dans des concentrations de l'ordre de 0,6 g/kg de mélange réactionnel, ce qui représente un rendement global de l'ordre de 10%.

L'invention est illustrée de manière plus détaillée par les exemples suivants dans lesquels les températures sont indiquées en degrés centigrades et les abréviations ont le sens usuel dans l'art.

### Manières de réaliser l'invention

### Exemple 1

### Préparation de n-hexanal et n-hexanol

Dans un réacteur 4 cols équipé d'un agitateur mécanique, d'un thermomètre, d'une ampoule à introduction et d'une électrode pH, on a dissous 315,3 g d'une solution d'hydrolysat d'huile de tournesol dans 1048 g d'eau et la valeur du pH a été ajustée à 9,5. La température du mélange a été baissée à 10°C, puis le mélange a été dégazé avant d'y introduire un courant d'oxygène.

132 g de farine de soja fraîchement moulue ont été ajoutés au mélange réactionnel tandis que la vitesse d'agitation a été portée à 1000 tpm.

Après 1 h environ, l'absorption d'oxygène cesse et une titration iodométrique indique un degré d'oxydation d'environ 92%.

La solution résultante, contenant le "C₁₃HDPO", a été transférée dans un ballon tricol de 6 l équipé d'un thermomètre, d'un agitateur et d'une électrode pH.

La valeur du pH a été ajustée à 8,5 et maintenue à cette valeur par l'adjonction d'une solution alcaline de NaOH à 10% pendant qu'on ajoutait 1400 g d'homogénat de goyave. Le mélange résultant a été maintenu sous agitation à 10°C pendant 30 min, puis la température a été augmentée à 25°C tandis que le déroulement de la réaction a été suivi par l'analyse périodique d'échantillons du mélange de réaction.

Le n-hexanal, présent à raison de 3,80 g/kg de mélange réactionnel, peut être isolé à ce stade du procédé par un entraînement à la vapeur et séparation de la phase organique suivie de sa distillation.

Le procédé peut être poursuivi jusqu'à conversion complète de l'hexanal formé en n-hexanol en opérant de la manière suivante.

600 g de levure boulangère ont été ajoutés au mélange de réaction, suivis par deux autres fractions de 100 g chacune de cette même levure ajoutées dans un intervalle d'environ 21/2h.

Après disparition complète du n-hexanal, on a procédé à une distillation sous vide [T=78°, P=460 mbar].

On a ainsi recueilli une fraction de 800 ml de distillat qui a été extraite avec 3 fractions de 300 ml chacune d'éther. Les extraits organiques combinés ont été concentrés et le résidu obtenu a été distillé à 12 mbar pour donner 13,75 g de n-hexanol dont la pureté était de 96%.

Le tableau ci-dessous résume les résultats obtenus au cours de différents essais effectués en variant les concentrations d'homogénat de goyage et de levure ainsi que le pH.

**TABLEAU**

| Ex. | C₁₃HDPO /goyave | Conc. goyave | Conc. levure boulangère | | | Rendement n-hexanol | |
|---|---|---|---|---|---|---|---|
| | g/kg ²⁾ | g/kg ¹⁾ | g/kg ¹⁾ | pH | Temp.°C | g/kg ¹⁾ | rend. global % |
| 1 | 37,7 | 500 | 75 | 5 | 25 | 0,60 | 10,7 |
| 2 | 37,7 | 500 | 150 | 5 | 10 | 2,43 | 45,4 |
| 3 | 43,8 | 493 | 150 | 5 | 25 | 2,72 | 47,7 |
| 4 | 43,1 | 540 | 162 | 5 | 25 | 2,98 | 45,3 |
| 5 | 51,9 | 540 | 162 | 5 | 25 | 3,23 | 49,1 |
| 6 | 47,3 | 595 | 162 | 5 | 25 | 2,34 | 38,9 |
| 7 | 51,9 | 541 | 216 | 5 | 25 | 2,71 | 43,2 |
| 8 | 52,1 | 571 | 171 | 8,5 | 25 | 2,98 | 36,0 |
| 9 | 74,4 | 483 | 276 | 8,5 | 25 | 4,12 | 44,9 |
| 10 | 74,4 | 483 | 241 | 8,5 | 25 | 3,72 | 39,4 |
| 11 | 74,4 | 483 | 207 | 8,5 | 25 | 3,65 | 37,6 |
| 12 | 74,4 | 483 | 276 | 8,5 | 25 | 3,81 | 41,5 |

| | | | | | | | |
|---|---|---|---|---|---|---|---|
| 1) rapport poids en g, par kg de mélange de réaction | | | | | | | |
| 2) rapport pondéral par rapport au poids de goyave | | | | | | | |

Des résultats analogues ont été observés en remplaçant l'hydrolysat d'huile de tournesol par un mélange d'acides gras du commerce (origine: Fluka AG, Suisse) dont le contenu en acide linoléique était d'environ 55%.

A titre d'agent de lipoxygénase on peut également remplacer la farine de soja en poudre par un homogénat obtenu comme suit.

On traite successivement de la farine de soja avec deux fractions d'hexane puis on mélange la partie solide ainsi dégraissée avec un poids identique d'une solution de borax 0,05 M (5 min/4°C) et on centrifuge (5 min/1000 tpm/5°C). La solution ainsi débarrassée des parties solides a été utilisée comme agent de lipoxygénase.

La levure boulangère employée était une levure du commerce (origine : Hefefabrik Hindelbank, Suisse).

### Exemple 2

### Préparation de n-hexanal

Dans un réacteur 4 cols équipé d'un agitateur mécanique, d'un thermomètre, d'une ampoule d'introduction et d'une électrode pH, on a mélangé 169,2 g d'un mélange d'acides gras du commerce, dont le contenu en acide linoléique était d'environ 64,5%, 60 g d'une solution aqueuse de NaOH à 30% et 1048 g d'eau. La température du mélange a été ajustée à 18°C et le pH à 9,5, puis le mélange a été dégazé avant d'y introduire de l'oxygène.

132 g de farine de soja fraîchement moulue ont été ajoutés au mélange réactionnel tandis que le pH a été maintenu à 9,5 par addition graduelle d'une solution à 30% de NaOH. La réaction procède comme indiqué à l'exemple précédent. Une fois cessée l'absorption d'oxygène, la solution obtenue contenant 87,3 g/kg de "C13HDPO" a été transférée dans un autre réacteur et traitée avec un homogénat de goyave comme décrit à l'exemple 1.

L'hexanal a été séparé du mélange de réaction par distillation à 78°C et une pression de 460mbar. Une fraction d'environ 280g a été ainsi obtenue, fraction composée de deux couches dont la supérieure a été séparée et distillée à 15 mbar pour fournir 14,24 g de n-hexanal dont la pureté était de 98% et le rendement global de 35,8%.

La production de n-hexanal a pu ainsi être effectuée avec une concentration égale à 5,07 g d'aldéhyde par kg de mélange réactionnel.

### Exemple 3

### Préparation de 3-(Z)-hexén-1-ol

Dans un réacteur équipé d'un agitateur mécanique, d'un thermomètre, d'une ampoule à introduction et d'une électrode pH, on a agité 197,5g d'un mélange d'origine commerciale contenant 48,2% d'acide linolénique (le reste étant constitué par de l'acide linoléique et oléique), 60,5 g de NaOH à 30% et 930 ml d'eau. La température a été ajustée à 20°C et le pH a été porté à 9,5.

Après avoir dégazé, on a introduit de l'oxygène, puis 174 g de farine de soja ont été additionnés au mélange réactionnel, tandis que la température du mélange a été maintenue à 18-22°C par refroidissement extérieur et le pH gardé constant à 9,5 par l'addition graduelle d'une solution de NaOH à 30%. Après environ 1 h, l'absorption d'oxygène cesse et une titration iodométrique indique un degré d'oxydation de 97%.

La solution résultante, contenant le "C₁₃ HDPOT", a été transférée dans un ballon tricol de 6 l équipé d'un thermomètre, d'un agitateur et d'une électrode pH. La température de la solution a été portée à 23°C, tandis que le pH a été maintenu à 8,0 pendant qu'on ajoutait 900 g de levure boulangère suivie, une minute plus tard par 1500 g d'homogénat de goyave. La température a été augmentée à 25°C et le déroulement de la réaction a été suivi par l'analyse périodique d'échantillons du mélange de réaction. Après disparition complète de 3-(Z)-hexén-1-al, on a procédé à une distillation sous vide [T = 78°; P = 460 mbar].

Une fraction de 760 g a été ainsi recueillie et extraite avec 3 fractions d'acétate d'éthyle. Les extraits organiques combinés ont fourni après évaporation et distillation 17,2 g d'une huile brute qui par fractionnement à 15 mbar ont donné 14,36 g de 3-(Z)-hexén-1-ol dont la pureté était de 88%. Le rendement global en hexénol a été de 36,9% ou 3,79 g par kg de mélange réactionnel.

Plusieurs essais ont été effectués pour les étapes (b) et (c) en variant la valeur du pH. Le graphique ci-dessous résume les résultats obtenus en indiquant la concentration en pourcentage relatif de 3-(Z)-hexén-1-ol.

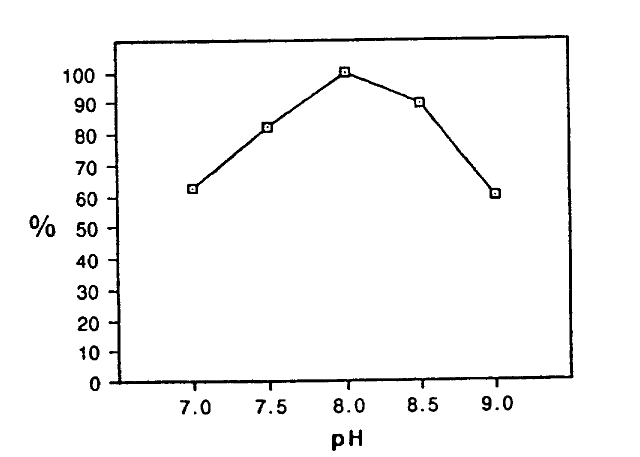

En suivant le même mode opératoire que celui décrit ci-dessus, on peut transformer de manière similaire de l'huile de lin hydrolysé au lieu de l'acide linolénique. A cet effet on a employé un hydrolysat préparé à partir d'une huile de lin commerciale. L'hydrolysat a été préparé de la manière suivante. 103 g d'huile brute ont été mélangés avec 58,0 g d'une solution aqueuse à 30% de NaOH et 80 g d'éthanol, et le tout a été chauffé à reflux sous agitation. Après refroidissement la solution d'acides gras obtenue [56,4% d'acide linolénique, 16,8% d'acide linoléique et 16,5% d'acide oléique] solidifie sous forme d'un gel.

Selon une variante de la méthode décrite ci-dessus on a préparé un hydrolysat d'huile de lin de la façon suivante.

On a chauffé à reflux pendant 1 h un mélange de 518,3 g d'huile de lin, 290 g d'une solution aqueuse à 30% de NaOH et 400 g de propylène glycol. Le gel obtenu après refroidissement est utilisé directement pour l'étape suivante de réaction. Le taux d'acide linolénique dans le mélange d'acides gras formé était similaire à celui observé dans le mélange obtenu suivant la méthode précédente.

A titre d'agent de lipoxygénase on peut également remplàcer la farine de soja en poudre par un homogénat obtenu comme suit.

On traite successivement de la farine de soja avec deux fractions d'hexane puis on mélange la partie solide ainsi dégraissée avec un poids identique d'une solution de borax 0,05 M (5 min/4°C) et on centrifuge (5 min/1000 tpm/5°C). La solution ainsi débarrassée des parties solides a été utilisée comme agent de lipoxygénase.

La levure boulangère employée était une levure du commerce [origine : Hefefabrik Hindelbank, Suisse].

Le tableau qui suit résume les résultats obtenus au cours de différents essais effectués en variant les concentrations d'homogénat de goyave et de levure.

| | Produit de départ | C₁₃HDPOT /goyave | Conc. goyave | Levure boulangère | Rendement 3-(Z)-hexén-1-ol | |
|---|---|---|---|---|---|---|
| Ex. | 1) 3) | g/kg | g/kg ²⁾ | conc. g/kg ²⁾ | g/kg ²⁾ | rend. global 4) |
| 1 | (a) | 71,2 | 484 | 207 | 2,25 | 25,5% |
| 2 | (b) | 69,9 | 515 | 221 | 2,58 | 31,8% |
| 3 | (b) | 87,5 | 527 | 174 | 2,84 | 25,8% |
| 4 | (b) | 87,3 | 527 | 208 | 3,36 | 33,7% |
| 5 | (b) | 97,7 | 519 | 237 | 3,79 | 36,9% |
| 6 | (b) | 99,7 | 519 | 237 | 3,68 | 36,5% |
| 7 | (b) | 99,2 | 519 | 237 | 3,57 | 34,9% |
| 8 | (b) | 96,3 | 519 | 216 | 4,20 | 41,9% |
| 9 | (b) | 98,4 | 510 | 203 | 3,72 | 35,4% |
| 10 | (b) | 73,1 | 515 | 181 | 2,49 | 31,1% |
| 11 | (b) | 73,6 | 515 | 181 | 2,47 | 29,2% |
| 12 | (b) | 101,4 | 527 | 228 | 3,66 | 32,4% |

| | | | | | | |
|---|---|---|---|---|---|---|
| 1) (a) = hydrolysat d'huile de lin (b) = mélange d'acides gras [48,2% acide linolénique] | | | | | | |
| 2) poids en g par rapport à 1 kg du mélange de réaction | | | | | | |
| 3) rapport pondéral constant de farine de soja/produit de départ | | | | | | |
| 4) compte tenu de la pureté | | | | | | |

### Exemple 4

### Préparation de 2-(E)-hexén-1-al

Dans un réacteur 5 cols équipé d'un agitateur mécanique, d'un thermomètre, d'une ampoule à introduction et d'une électrode pH, on a dissous 315,3 g d'une solution d'hydrolysat d'huile de lin dans 1248 g d'eau et agité à 20°C. La valeur du pH a été ajustée à 9,5 et le mélange a été dégazé avant d'y introduire un courant d'oxygène.

132 g de farine de soja fraîchement moulue ont été ajoutés au'mélange réactionnel tandis que la vitesse d'agitation a été portée à 1000 tpm. La température a été maintenue entre 18 et 22°C à l'aide d'un bain d'eau froide. Après 1 h environ, l'absorption d'oxygène cesse et une titration iodométrique indique un degré d'oxydation d'environ 95%.

La solution résultante, contenant le "C₁₃HDPOT" a été transférée dans un ballon tricol de 6 l équipé d'un thermomètre, d'un agitateur et d'une électrode pH.

La température a été ajustée à 5°C et 2 kg d'homogénat de goyave, refroidi à 5°C, ont été ajoutés sous agitation vigoureuse. Le mélange résultant a été maintenu sous agitation à cette température pendant 15 min, le pH mesurant 7,3.5 g d'acide citrique ont été ajoutés en une fois et le pH est descendu à 6,5.

L'électrode a ensuite été remplacée par un pont de distillation et le mélange a été distillé à 78°C (P=460 mbar).

Une fraction de 700 g (environ 20% du volume de réaction total) ont été condensés et extraits avec 3 fractions de 150 ml chacune d'éther. Les extraits organiques combinés ont été séchés sur Na₂SO₄ et concentrés. Le résidu obtenu (7,4 g) a été distillé à 30 mbar pour fournir une fraction de 6,72 g de 2-(E)-hexén-1-al dont la pureté était de 79%.

### Exemple 5

### Préparation de 2-(E)-hexén-1-ol

Dans un réacteur 5 cols équipé d'un agitateur mécanique, d'un thermomètre, d'une ampoule d'introduction et d'une électrode pH, on a mélangé 134,9 g d'un mélange d'acides gras du commerce, dont le contenu en acide linolénique était d'environ 48,2%, 41 g d'une solution aqueuse de NaOH à 30% et 1248 g d'eau. La température du mélange a été ajustée à 20°C et le pH à 9,5, puis le mélange a été dégazé avant d'y introduire de l'oxygène.

132 g de farine de soja fraîchement moulue ont été ajoutés au mélange réactionnel tandis que le pH a été maintenu à 9,5 par addition graduelle d'une solution à 30% de NaOH. La réaction procède comme indiqué à l'exemple précédent. Une fois cessée l'absorption d'oxygène, la solution obtenue contenant le "C₁₃HDPOT" a été transférée dans un autre réacteur et traitée avec un homogénat de goyave comme décrit à l'exemple 1.

On a ajouté 5 g d'acide citrique et chauffé à 50°C, l'isomérisation du 3-(Z)-hexén-1-al en 2-(E)-hexén-1-al étant suivie par GC. Après 90 min, la transformation était complète et la température de la réaction a été baissée à 25°C.

On a ajouté 500 g de levure boulangère et suivi la réaction en retirant des échantillons pour analyse toutes les 30 min. Après 60 min, 85% de l'aldéhyde avaient été réduits en alcool.

L'électrode a été remplacée par le pont de distillation et le mélange distillé à 78°C (P=460 mbar).

Une fraction de 800 g a été condensée et extraite comme décrit à l'exemple 1.

La distillation du résidu sous une pression de 30 mbar, a fourni 3,76 g de 2-(E)-hexén-1-ol dont la pureté était de 63%.

## Revendications

1. Procédé enzymatique pour la préparation de n-hexanal, 3-(Z)-hexén-1-al, ou 2-(E)-hexén-1-al, ou des alcools correspondants, à partir de l'acide linoléique, de l'acide linolénique, ou d'un précurseur d'origine naturelle de l'un ou l'autre acide sous forme d'hydrolysat d'huile végétale, caractérisé en ce qu'on effectue les étapes réactionnelles distinctes suivantes:
a) on soumet soit l'acide linoléique, soit l'acide linolénique, ou ledit précurseur sous forme d'hydrolysat d'huile végétale, à l'action d'une lipoxygénase du soja dans un milieu réactionnel approprié, pour obtenir soit l'acide 13-hydropéroxy-octadéca-9,11-diénoïque ("C₁₃HDPO"), soit l'acide 13-hydropéroxy-octadéca-9,11,15-triénoïque ("C₁₃HDPOT") ;
b) on transforme soit le "C₁₃HDPO", soit le "C₁₃HDPOT", ainsi formé soit en n-hexanal, soit en 3-(Z)-hexén-1-al, à l'aide d'une lyase de la goyave ;
c) on réduit soit le n-hexanal, soit le 3-(Z)-hexén-1-al, obtenu au moyen d'une levure pour fournir soit le n-hexanol, soit le 3-(Z)-hexén-1-ol ;
ou
c') le cas échéant, on isomérise le 3-(Z)-hexén-1-al dans les conditions de température et pH appropriées pour obtenir le 2-(E)-hexén-1-al, et
d) on réduit le 2-(E)-hexén-1-al obtenu au moyen d'une levure pour fournir le 2-(E)-hexén-1-ol.

2. Procédé selon la revendication 1, caractérisé en ce qu'il comprend l'étape supplémentaire de séparation du 2-(E)-hexén-1-al obtenu au moyen d'un entraînement à la vapeur d'eau suivi d'une extraction du produit désiré à l'aide d'un solvant organique inerte.

3. Procédé suivant la revendication 1, caractérisé en ce qu'il comprend l'étape supplémentaire de séparation du n-hexanal obtenu au moyen d'un entraînement à la vapeur d'eau suivi d'une séparation de la couche organique du distillat et de distillation.

4. Procédé selon la revendication 1, caractérisé en ce qu'il comprend l'étape supplémentaire de séparation du 3-(Z)-hexén-1-al obtenu par extraction du produit désiré à l'aide d'un solvant organique inerte.

5. Procédé selon la revendication 1, caractérisé en ce qu'il comprend l'étape supplémentaire de séparation du n-hexanol, du 3-(Z)-hexén-1-ol ou du 2-(E)-hexén-1-ol obtenu au moyen d'un entraînement à la vapeur d'eau suivi d'une extraction du produit désiré à l'aide d'un solvant organique inerte.

6. Procédé selon la revendication 1, caractérisé en ce que l'action de lipoxygénase est exercée par la farine de soja, laquelle est ajoutée au milieu réactionnel sous atmosphère d'oxygène.

7. Procédé selon la revendication 6, caractérisé en ce que la farine de soja est ajoutée à un milieu réactionnel dont le pH est maintenu à une valeur comprise entre 9,0 et 9,5.

8. Procédé selon la revendication 1, caractérisé en ce que l'étape b) est effectuée par l'action d'un homogénat de goyave.

9. Procédé selon la revendication 1, caractérisé en ce que l'étape c) ou d) est effectuée par l'action de levure boulangère.

10. Procédé selon la revendication 1, caractérisé en ce que les étapes b) et c) sont effectuées par l'action combinée d'un homogénat de goyave et de levure boulangère.

11. Procédé selon la revendication 1, caractérisé en ce que l'étape c') est effectuée à une température d'environ 50°C et un pH autour de 6,5.

12. Procédé selon la revendication 8, caractérisé en ce que la réaction s'effectue à un pH compris entre 7,0 et 9,0.

## Patentansprüche

1. Enzymatisches Verfahren zur Herstellung von n-Hexanal, 3-(Z)-Hexen-1-al oder 2-(E)-Hexen-1-al oder der entsprechenden Alkohole aus Linolsäure oder Linolensäure oder einem Präkursor natürlicher Herkunft der einen oder der anderen Säure in Form eines Pflanzenöl-Hydrolysats, dadurch gekennzeichnet, dass man die folgenden unterschiedlichen Reaktionsstufen durchführt:
a) man unterwirft die Linolsäure oder die Linolensäure oder deren Präkursor in Form eines Pflanzenöl-Hydrolysats der Einwirkung einer Soja-Lipoxygenase in einem geeigneten Reaktionsmedium, um die 13-Hydroperoxy-octadeca-9,11-diensäure ("C₁₃HDPO") oder die 13-Hydroperoxy-octadeca-9,11,15-triensäure ("C₁₃HDPOT") zu erhalten;
b) man setzt das so gebildete "C₁₃HDPO" oder das "C₁₃HDPOT" mit Hilfe einer Lyase der Guave entweder zu dem n-Hexanal oder zu dem 3-(Z)-Hexen-1-al um;
c) man reduziert das erhaltene n-Hexanal oder 3-(Z)-Hexen-1-al mittels einer Hefe,um das n-Hexanol oder das 3-(Z)-Hexen-1-ol zu erhalten;
oder
c') man isomerisiert gegebenenfalls das 3-(Z)-Hexen-1-al unter geeigneten Temperatur- und pH-Bedingungen, um das 2-(E)-Hexen-1-al zu erhalten, und
d) man reduziert das erhaltene 2-(E)-Hexen-1-al mittels einer Hefe, um das 2-(E)-Hexen-1-ol zu erhalten.

2. Verfahren gemäss Patentanspruch 1, dadurch gekennzeichnet, dass es die zusätzliche Stufe der Abtrennung des erhaltenen 2-(E)-Hexen-1-als durch Wasserdampfdestillation, gefolgt von einer Extraktion des gewünschten Produktes mit Hilfe eines inerten organischen Lösungsmittels, umfasst.

3. Verfahren gemäss Patentanspruch 1, dadurch gekennzeichnet, dass es die zusätzliche Stufe der Abtrennung des erhaltenen n-Hexanals mittels Wasserdampfdestillation, gefolgt von einer Abtrennung der organischen Schicht des Destillats und Destillation, umfasst.

4. Verfahren gemäss Patentanspruch 1, dadurch gekennzeichnet, dass es die zusätzliche Stufe der Abtrennung des erhaltenen 3-(Z)-Hexen-1-als mittels Extraktion des gewünschten Produktes mit Hilfe eines inerten organischen Lösungsmittels umfasst.

5. Verfahren gemäss Patentanspruch 1, dadurch gekennzeichnet, dass es die zusätzliche Stufe der Abtrennung des erhaltenen n-Hexanol, 3-(Z)-Hexen-1-ol oder 2-(E)-Hexen-1-ol mittels Wasserdampfdestillation, gefolgt von einer Extraktion des gewünschten Produktes mit Hilfe eines inerten organischen Lösungsmittels, umfasst.

6. Verfahren gemäss Patentanspruch 1, dadurch gekennzeichnet, dass die Wirkung der Lipoxygenase durch Sojamehl erhalten wird, welches dem Reaktionsmedium in einer Sauerstoffatmosphäre zugesetzt wird.

7. Verfahren gemäss Patentanspruch 6, dadurch gekennzeichnet, dass das Sojamehl einem Reaktionsmedium zugesetzt wird, dessen pH auf einem Wert zwischen 9,0 und 9,5 gehalten wird.

8. Verfahren gemäss Patentanspruch 1, dadurch gekennzeichnet, dass die Stufe b) durch Einwirkung eines Homogenisats von Guave erfolgt.

9. Verfahren gemäss Patentanspruch 1, dadurch gekennzeichnet, dass die Stufe c)oder d) durch Einwirkung von Bäckerhefe erfolgt.

10. Verfahren gemäss Patentanspruch 1, dadurch gekennzeichnet, dass die Stufen b) und c) durch kombinierte Einwirkung eines Homogenisats von Guave und Bäckerhefe erfolgen.

11. Verfahren gemäss Patentanspruch 1, dadurch gekennzeichnet, dass die Stufe c') bei einer Temperatur von ca. 50°C und einem pH im Bereich von 6,5 erfolgt.

12. Verfahren gemäss Patentanspruch 8, dadurch gekennzeichnet, dass die Reaktion bei einem pH zwischen 7,0 und 9,0 erfolgt.

## Claims

1. Enzymatic process for the preparation of n-hexanal, 3(Z)-hexen-1-al or 2-(E)-hexen-1-al, or of their corresponding alcohols, starting from linoleic acid or linolenic acid, or from a natural origin precursor of said one or the other acid in the form of a vegetable oil hydrolysate, characterized in that the following discrete reaction steps are carried out :
a) linoleic acid, respectively linolenic acid, or said precursor in the form of a vegetable oil hydrolysate, is subjected to the action of a soya lipoxygenase in an appropriate reaction medium to obtain 13-hydroperoxy-octadeca-9,11-dienoic acid ("C₁₃HDPO"), respectively 13-hydroperoxy-octadeca-9,11,15-trienoic acid ("C₁₃HDPOT") ;
b) "C₁₃HDPO", respectively "C₁₃HDPOT", is converted into n-hexanal, respectively into 3-(Z)-hexen-1-al, by means of a guava lyase ;
c) the obtained n-hexanal, respectively 3-(Z)-hexen-1-al, is reduced by means of a yeast to obtain n-hexanol, respectively 3-(Z)-hexen-1-ol ;
or
c') if necessary, 3-(Z)-hexen-1-al is isomerised under the appropriate temperature and pH conditions to obtain 2-(E)-hexen-1-al, and
d) the obtained 2-(E)-hexen-1-al is reduced by means of a yeast to provide 2-(E)-hexen-1-ol.

2. Process according to claim 1, characterized in that it comprises the additional step of separating the obtained 2-(E)-hexen-1-al by way of steam distillation followed by the extraction of the desired product by means of an inert organic solvent.

3. Process according to claim 1, characterized in that it comprises the additional step of separating the obtained n-hexanal by way of steam distillation followed by a separation of the distillate's organic layer and by distillation.

4. Process according to claim 1, characterized in that it comprises the additional step of separating the obtained 3-(Z)-hexen-1-al through extraction of the desired product by means of an inert organic solvent.

5. Process according to claim 1, characterized in that it comprises the additional step of separating the obtained n-hexanol, 3-(Z)-hexen-1-ol or 2-(E)-hexen-1-ol by way of steam distillation followed by extraction of the desired product by means of an inert organic solvent.

6. Process according to claim 1, characterized in that the lipoxygenase action is carried out by soya flour which is added to the reaction medium under oxygen atmosphere.

7. Process according to claim 6, characterized in that the soya flour is added to a reaction medium the pH of which is maintained at a value comprised between 9.0 and 9.5.

8. Process according to claim 1, characterized in that step b) is carried out via the action of a guava homogenate.

9. Process according to claim 1, characterized in that step c) or d) is carried out via the action of baker's yeast.

10. Process according to claim 1, characterized in that steps b) and c) are carried out via the combined action of a guava homogenate and baker's yeast.

11. Process according to claim 1, characterized in that step c') is carried out at a temperature of about 50°C and pH around 6.5.

12. Process according to claim 8, characterized in that the reaction is carried out at a pH value comprised between 7.0 and 9.0.
